# EUROPÄISCHE PATENTSCHRIFT

(11) **EP 4 235 161 B1**
(45) Veröffentlichungstag und Bekanntmachung des Hinweises auf die Patenterteilung: **08.04.2026**
(21) Anmeldenummer: 23157559.8
(22) Anmeldetag: 20.02.2023
(51) Int. Cl.: G01N 25/48, G01N 5/04, G01N 35/10

(54) **VORRICHTUNG UND VERFAHREN FÜR EINE THERMISCHE ANALYSE EINER PROBE**
DEVICE AND METHOD FOR THERMAL ANALYSIS OF A SAMPLE
DISPOSITIF ET PROCÉDÉ D'ANALYSE THERMIQUE D'UN ÉCHANTILLON

(30) Priorität: 24.02.2022 DE 102022104400
(43) Veröffentlichungstag der Anmeldung: 30.08.2023
(73) Patentinhaber: Netzsch-Gerätebau GmbH, 95100 Selb (DE)
(72) Erfinder: Füglein, Dr., Ekkehard, 95100 Selb (DE); Neumann, Georg, 95173 Schönwald (DE)
(74) Vertreter: Roos, Peter

(56) Entgegenhaltungen:
- DE-A1- 102014 004 701
- DE-A1- 19 836 372
- JP-A- 2013 186 090
- US-A1- 2003 199 099

## Beschreibung

Die vorliegende Erfindung betrifft eine Vorrichtung sowie ein Verfahren für eine thermische Analyse einer Probe.

Vorrichtungen zur thermischen Analyse einer Probe sind in vielfältigen Ausführungen aus dem Stand der Technik bekannt und weisen eine Probenkammer zur Aufnahme (wenigstens) einer zu analysierenden Probe oder zur Aufnahme (wenigstens) eines Probentiegels auf, in dessen Inneren sich die zu analysierende Probe befindet. Des Weiteren weisen die Vorrichtungen eine Temperiereinrichtung zum Temperieren der Probenkammer sowie eine Messeinrichtung zur Messung einer Temperatur der Probe und einer oder mehrerer weiterer Messgrößen auf.

Verfahren zur thermischen Analyse können vorteilhaft z. B. zur Charakterisierung von Materialeigenschaften (z. B. spezifische Wärmekapazität, Wärmeleitfähigkeit, Schmelz-, Zersetzungs- und Kristallisationstemperaturen) eingesetzt werden, einschließlich z. B. der Eigenschaften der temperaturabhängig bzw. bei Temperaturveränderung stattfindenden Prozesse (z. B. Enthalpien bei Phasenübergängen oder chemischen Reaktionen etc.).

Beispiele für Methoden zur thermischen Analyse sind z. B. die Thermogravimetrie (TG), die Differentialthermoanalyse (DTA) sowie die daraus abgeleitete Methode der Dynamischen Differenzkalorimetrie ("Differential Scanning Calorimetry", DSC).

Bei den im Rahmen der Erfindung besonders interessierenden Verfahren wird die Probe typischerweise gemäß eines Temperaturprogramms temperiert, in dessen Verlauf eine Kammertemperatur im Inneren der Probenkammer verändert und gleichzeitig mittels der Messeinrichtung die Probentemperatur und zusätzlich eine oder mehrere weitere Messgrößen der Probe betreffend Eigenschaften der Probe gemessen werden.

Bei der Thermogravimetrie (TG) wird als eine solche "Eigenschaft der Probe" beispielsweise das Gewicht bzw. die Masse der Probe im Verlauf eines Temperaturprogramms gemessen.

Je nach konkreter Ausführung der Messeinrichtung kann bei der thermischen Analyse neben einer Messung der Probentemperatur und z. B. der Probenmasse prinzipiell eine Vielzahl von Messungen weiterer physikalischer Größen im Verlauf des Temperaturprogramms vorgesehen sein.

Ein Beispiel hierfür ist die Ermittlung der temperaturabhängigen kalorischen Effekte einer Probe mittels DSC mit simultaner Ermittlung der temperaturabhängigen Masse der Probe mittels Thermogravimetrie (TG). Ein weiteres Beispiel ist die Ermittlung der Art und Menge an flüchtigen Zersetzungsprodukten einer Probe im Verlauf des Temperaturprogramms mittels Massenspektroskopie (MS) mit simultaner Ermittlung der temperaturabhängigen Masse der Probe mittels Thermogravimetrie (TG). Eine Thermische Analyse unter gleichzeitigem Einsatz mehrerer verschiedener Methoden sei hier auch als "Simultane Thermische Analyse" bezeichnet.

Der hier verwendete Begriff der (von der Messeinrichtung gemessenen) "Eigenschaften der Probe" ist insofern also sehr weitgehend zu verstehen und kann z. B. die vorerwähnte "Art und Menge" an flüchtigen Zersetzungsprodukten umfassen.

Bei bestimmten Materialien und daraus hergestellten Proben ergibt sich das Problem, dass die Proben durch Bestandteile der Atmosphäre wie z. B. Sauerstoff, Kohlendioxid, Wasser (Luftfeuchtigkeit) etc. kontaminiert werden können und/oder mit derartigen Bestandteilen chemisch reagieren können, so dass die betreffenden Proben hierdurch in undefinierter Weise verändert werden.

Im Hinblick auf Lagerung und/oder Transport einer Probe besteht eine einfache Lösung des Problems darin, die Probe unmittelbar nach deren Herstellung in einem Probentiegel mit einer darauf aufgesetzten luftdichten Tiegelabdeckung zu "verpacken" oder die Probe in einem Probentiegel herzustellen und diesen Tiegel hierbei sogleich mit der luftdichten Tiegelabdeckung zu versehen.

Auch bei einer in einem Probentiegel mit einer darauf aufgesetzten luftdichten Tiegelabdeckung befindlichen Probe verbleibt jedoch die vorerwähnte Gefahr der Kontamination und/oder chemischen Reaktion, sobald die Probe im Rahmen einer sogenannten "Probenvorbereitung" in die Vorrichtung eingebracht und hierbei in Vorbereitung der thermischen Analyse die Tiegelabdeckung entfernt oder anderweitig geöffnet werden muss, was bei vielen thermischen Analysen zwingend notwendig ist.

Eine in Betracht kommende Lösung dieses Problems ist es, den noch luftdicht verschlossenen Probentiegel mitsamt der Vorrichtung in eine luftdicht verschlossene Kammer (sog. "glove box") einzubringen und sodann die Probenvorbereitung und Durchführung der thermischen Analyse in dieser Kammer unter Vakuum oder unter einer Schutzgasatmosphäre durchzuführen.

Abgesehen von dieser sehr aufwendigen und aus Kostengründen eher unpraktikablen Lösung lässt sich das Problem unter Verwendung eines Probentiegels mit einer Tiegelabdeckung etwas abmildern, welche bei der Probenvorbereitung nicht vollständig abgenommen (z. B. abgeschraubt) werden muss, sondern bei der z. B. mit einer Nadel ein nur kleines Loch eingestochen werden kann, um somit für die nachfolgende thermische Analyse eine mediumdurchlässige Verbindung zwischen dem Inneren des Probentiegels und der Probenkammer der Vorrichtung zu schaffen.

Jedoch auch bei dieser Vorgehensweise, d. h. dem "Aufstechen" der Tiegelabdeckung in Umgebungsluft, verbleibt eine gewisse Gefahr der Kontamination und/oder chemischen Reaktion mit Bestandteilen der Umgebungsluft während des Einbringens der Probe in die Vorrichtung.

In der DE 198 36 372 A1 ist eine Heizeinheit zur Verdampfung einer Probe durch Erhitzung beschrieben. Die Probe befindet sich dabei in einem Behälter, dessen Öffnung hermetisch durch eine Gummidichtung abgeschlossen ist, wobei die Heizeinheit hohlnadelartig ausgebildete Einlassrohre zum Zuführen von Trägergas in den Behälter und ein hohlnadelartig ausgebildetes Auslassrohr zum Ausstoßen der verdampften Bestandteile und des Trägergases aus dem Behälter umfasst, welche die Gummidichtung während des Einbringens des Behälters in die Heizeinheit durchstoßen.

In der JP 2013-186090 A ist ein Kalorimeter mit zwei in einem Heizbereich ausgebildeten Aufnahmeabschnitten zur Aufnahme eines jeweiligen Probenbehälters beschrieben. Mündungen der Aufnahmeabschnitte sind jeweils mit einem Stopfen verschlossen und die Probenbehälter sind jeweils mit einem Schraubdeckel verschlossen, wobei eine jeweilige hohle Nadel durch den Stopfen und den Schraubdeckel hindurch verläuft, so dass Gase aus dem jeweiligen Probenbehälter über die Nadel ausgelassen werden können.

Es ist eine Aufgabe der vorliegenden Erfindung, bei einer Vorrichtung und einem Verfahren für eine thermischen Analyse die im Rahmen einer Probenvorbereitung bestehende Gefahr einer Veränderung der Probe infolge unerwünschter chemischer oder physikalischer Reaktionen zu verringern.

Gemäß eines ersten Aspekts der Erfindung wird diese Aufgabe durch eine Vorrichtung für eine thermische Analyse einer Probe mit den Merkmalen des Anspruchs 1 gelöst.

Mit der Erfindung ist es vorteilhaft möglich, die Gefahr einer unerwünschten Veränderung der Probe infolge chemischer oder physikalischer Reaktionen mit Bestandteilen der Umgebungsluft drastisch zu verringern, da der Probentiegel in luftdicht verschlossenem Zustand in die Probenkammer eingebracht und dort unter einer definierten Gasatmosphäre geöffnet werden kann.

In einer bevorzugten Ausführungsform der Erfindung ist vorgesehen, dass die in der Probenkammer erzeugte Gasatmosphäre wenigstens ein Gas enthält, das ausgewählt ist aus der Gruppe bestehend aus Stickstoff, Argon und Helium. Eine derartige Gasatmosphäre kann im Hinblick auf deren Zweck auch als Inertgasatmosphäre oder Schutzgasatmosphäre bezeichnet werden.

Bei vielen herkömmlichen Vorrichtungen und Verfahren der thermischen Analyse ist ohnehin vorgesehen, dass während der Durchführung der thermischen Analyse die Probenkammer von einem sogenannten Trägergas durchströmt wird, um von der Probe freigesetzte gasförmige Stoffe (z. B. flüchtige Bestandteile oder flüchtige Reaktionsprodukte) in definierter Weise abzuführen und einer Analyseeinheit der Messvorrichtung wie z. B. einer Gaschromatographie/Massenspektroskopie-Einrichtung zuzuführen.

In diesem Fall kann vorgesehen sein, dass zumindest ein Teil (z. B. Gasquelle, Gaseinlass etc.) der Mittel zur Erzeugung einer Trägergasströmung in der Probenkammer zusätzlich zur Erzeugung der Gasatmosphäre in der Probenkammer im Rahmen der Probenvorbereitung genutzt wird. Die Zusammensetzungen der Gase einerseits bei der Probenvorbereitung ("Schutzgas") und andererseits bei der Durchführung der thermischen Analyse ("Trägergas") können identisch oder aber voneinander verschieden gewählt sein.

In einer Ausführungsform der Erfindung ist vorgesehen, dass die Kammerabdeckung und die Stecheinrichtung als eine Abdeckungs/Stech-Einheit baulich zusammengefasst ausgebildet sind.

Damit ergibt sich für viele bereits im Einsatz befindliche Vorrichtungskonstruktionen der Vorteil, dass die Erfindung durch eine eher geringfügige Modifikation der Konstruktion und somit einfach und kostengünstig realisiert werden kann.

In einer Weiterbildung dieser Ausführungsform ist vorgesehen, dass die Vorrichtung eine weitere Kammerabdeckung aufweist, die anstelle der Abdeckungs/Stech-Einheit auf die Kammeröffnung der Probenkammer aufsetzbar ist.

Diese Weiterbildung ist z. B. insbesondere dann sehr vorteilhaft, wenn die Kammerabdeckung bei der Durchführung der thermischen Analyse abgesehen von der Funktion zum Schutz eines Austausches von Medien und/oder Energie (Wärme) zwischen Probenkammer und Umgebung noch wenigstens eine weitere Funktion besitzt. Gemäß dieser Weiterbildung kann nämlich nach dem Stechen des Lochs in die Tiegelabdeckung und vor der Durchführung der thermischen Analyse der Probe die Abdeckungs/Stech-Einheit durch eine hinsichtlich der genannten weiteren Funktion(en) optimierte Kammerabdeckung ersetzt werden. Eine solche weitere Funktion kann es z. B. sein, einen Gasauslass bereitzustellen, durch welchen hindurch bei der thermischen Analyse der Probe als Trägergas fungierendes Gas mitsamt von der Probe stammenden gasförmigen Komponenten aus der Probenkammer heraus zu einer einer Analyseeinheit der Messvorrichtung (z. B. Gaschromatographie/Massenspektroskopie-Einrichtung) weitergeleitet werden.

In einer anderen Weiterbildung dieser Ausführungsform ist vorgesehen, dass die Abdeckungs/Stech-Einheit einen Gasauslass (z. B. wenigstens eine kleine Öffnung) aufweist, durch den hindurch bei auf der Kammeröffnung aufgesetzter Abdeckungs/Stech-Einheit Gas aus der Gasatmosphäre der Probenkammer nach außen in die Umgebung strömen kann.

Mit dieser Weiterbildung kann unmittelbar nach dem Aufsetzen der Abdeckungs/Stech-Einheit vorteilhaft eine rasche und vollständige Verdrängung von Luft aus der Probenkammer erzielt werden, indem die Erzeugung der Gasatmosphäre durch eine mittels der Gasführungseinrichtung bewirkte Gasdurchströmung der Probenkammer realisiert wird, bei welcher Gas z. B. an einer relativ weit von der Kammeröffnung entfernten Stelle in die Probenkammer einströmen und an dem besagten Gasauslass der Abdeckungs/Stech-Einheit ausströmen gelassen wird.

Wenn in diesem Stadium der Probenvorbereitung (und hierbei bevorzugt weiter strömendem Gas) sodann das Loch in die Tiegelabdeckung gestochen wird und nachfolgend die Abdeckungs/Stech-Einheit von der Kammeröffnung der Probenkammer abgenommen wird (um diese vor der Durchführung der thermischen Analyse durch eine andere Kammerabdeckung zu ersetzen), so verhindert bzw. minimiert die Gasdurchströmung der Probenkammer in dieser Richtung, d.h. vom Inneren der Vorrichtung zur Kammeröffnung der Probenkammer, vorteilhaft ein Eindringen von Umgebungsluft in die Probenkammer.

Die Abdeckungs/Stech-Einheit kann z. B. eine zur Abdeckung der Kammeröffnung vorgesehene Wandung und ein an der Wandung ausgebildetes Führungslager zur bewegbaren Lagerung der Nadel aufweisen, wobei die Nadel sich von einer bei aufgesetzter Abdeckungs/Stech-Einheit der Probenkammer zugewandten Innenseite der Wandung durch die Wandung hindurch bis auf eine bei aufgesetzter Abdeckungs/Stech-Einheit der Probenkammer abgewandten Außenseite der Wandung erstrecken kann.

In letzterem Fall kann z. B. vorgesehen sein, dass ein für den Stechvorgang vorgesehener "Antrieb der Nadel" durch eine mechanische Einwirkung (Schub) auf den auf der Außenseite der Wandung befindlichen Abschnitt der Nadel bewerkstelligt wird. Dies besitzt z. B. den Vorteil, dass für den Antrieb der Nadel keine entsprechenden Antriebskomponenten innerhalb der Probenkammer erforderlich sind. Vielmehr können diese Antriebskomponenten ohne Beinflussung der Probenkammer und ohne Bauraumprobleme an der Außenseite der Abdeckungs/Stech-Einheit angeordnet sein.

Falls hierbei in der Verwendungssituation der Vorrichtung eine vertikale Stechbewegung der Nadel von oben nach unten vorgesehen ist, so kann in vielen Fällen insbesondere z. B. eine manuell bedienbare und somit sehr einfache Ausführungsform einer Abdeckungs/Stech-Einheit interessant sein, die eine zur Abdeckung der Kammeröffnung vorgesehene Wandung und ein an der Wandung ausgebildetes Führungslager zur bewegbaren Lagerung der Nadel aufweist, wobei die Nadel sich von der vorerwähnten Innenseite der Wandung durch die Wandung hindurch bis auf die vorerwähnte Außenseite der Wandung erstreckt, und wobei die Abdeckungs/Stech-Einheit ferner ein an der Abdeckungs/Stech-Einheit vertikalbeweglich geführtes Gewicht aufweist, das durch eine manuelle Bedienhandlung eines Benutzers aus einer vorbestimmten Höhe bezüglich der Nadel auf ein distales Ende der Nadel oder eine andere Stelle an der Nadel fallen gelassen werden kann.

Alternativ zu einer manuellen Bedienhandlung, mit der ein solches Gewicht auf die vorbestimmte Höhe angehoben und sodann fallengelassen werden kann, kommt auch in Betracht, die Stecheinrichtung bzw. die Abdeckungs/Stech-Einheit mit einer hierfür geeigneten motorischen Einrichtung auszustatten.

Alternativ zum Einsatz eines fallenden Gewichts, mit dem eine in der Regel vorteilhafte relativ rasche Einstechbewegung der Nadel bewirkt wird, kommt auch in Betracht, eine motorische Einrichtung vorzusehen, welche über eine Getriebeverbindung auf die Nadel einwirkt, um deren Einstechbewegung anzutreiben. Mit einer derartigen Ausführungsform ist es insbesondere auch möglich, eine relativ langsame Einstechbewegung der Nadel vorzusehen.

Eine optimale Einstechbewegung (z. B. rasch oder langsam, mit kurzem oder langem Hub), wie auch z. B. eine optimale Formgestaltung einer die Tiegelabdeckung einstechenden Nadel und hierbei insbesondere von deren Spitze, können abhängig vom Anwendungsfall (Konstruktion der Tiegelabdeckung) z. B. empirisch bestimmt werden.

In einer Ausführungsform besitzt die Nadel zumindest im Bereich ihrer Spitze und einem daran angrenzenden Schaftbereich eine rotationssymmetrische Form (z. B. zylindrischer Schaft mit konischer Spitze). Bei einer konischen Nadelspitze kann ein in Seitenansicht betrachtet von der Nadelspitze definierter Winkel z. B. in einem Bereich von 5° bis 60°, insbesondere 10° bis 30°, liegen.

In einer Ausführungsform der Erfindung ist vorgesehen, dass die Stecheinrichtung einen mechanischen Anschlag zur Begrenzung einer Einstechtiefe der Nadel aufweist. Alternativ oder zusätzlich kann z. B. vorgesehen sein, dass die Stecheinrichtung eine Federeinrichtung aufweist, welche die Nadel entgegen ihrer Stechrichtung vorbelastet, womit z. B. im Falle der vorerwähnten Ausführungsform mit einem fallenden Gewicht ebenfalls eine definierte Einstechtiefe erzielt werden kann.

In einer Ausführungsform der Erfindung ist vorgesehen, dass die Stecheinrichtung eine mechanische Zentriereinrichtung zur Zentrierung des Probentiegels samt Tiegelabdeckung bezüglich der Nadel aufweist.

Die Zentriereinrichtung kann z. B. zwei oder mehr Zentrierbacken zur Kontaktierung einer Mantelfläche des Probentiegels oder der darauf aufgesetzten Tiegelabdeckung aufweisen, um damit den Probentiegel samt Tiegelabdeckung in eine bestimmte Position bezüglich der Nadel zu zwingen (Zentrierung), bevor mittels der Nadel der Stecheinrichtung das Loch in die Tiegelabdeckung des Probentiegels gestochen wird.

In einer Ausführungsform der Erfindung ist vorgesehen, dass in der Probenkammer eine Tiegelaufnahme mit einer Abstellfläche zum darauf Abstellen des Probentiegels ausgebildet ist, wobei die Tiegelaufnahme verlagerbar ist zwischen einer ersten Stellung zum Abstellen des Probentiegels und Stechen eines Lochs in die Tiegelabdeckung und einer zweiten Stellung zum Durchführen der thermischen Analyse der Probe.

Die zweite Stellung der verlagerbaren Tiegelaufnahme ist hierbei bevorzugt weiter weg von der Kammeröffnung und/oder "weiter innen in der Vorrichtung" vorgesehen als die erste Stellung.

In einer spezielleren Ausführungsform ist eine Verlagerbarkeit der Tiegelaufnahme in Vertikalrichtung vorgesehen, wobei die zweite Stellung weiter weg von der Kammeröffnung und/oder "weiter innen in der Vorrichtung" als die erste Stellung vorgesehen ist.

Zwar sind verlagerbare Tiegelaufnahmen oftmals auch bei aus dem Stand der Technik bekannten Vorrichtungen vorgesehen, dienen dort jedoch in erster Linie nur dazu, ein Einbringen und Abstellen des z. B. mit einer Pinzette gehaltenen Probentiegels in die Vorrichtung (etwa durch eine relativ kleine Kammeröffnung der Probenkammer hindurch) zu vereinfachen, wobei zu bedenken ist, dass zur Gewährleistung einer möglichst präzisen Temperierung der zu analysierenden Probe in der Probenkammer die Probe sich während der nachfolgenden Analyse in der Regel in einer Position "weiter innen" in der Vorrichtung befinden muss.

Im Rahmen der vorliegenden Erfindung ermöglicht die vorerwähnte verlagerbare Tiegelaufnahme jedoch z. B. den zusätzlichen Vorteil, dass die konstruktive Realisierung der Stecheinrichtung, sei es mit der Kammerabdeckung baulich zusammengefasst (als Abdeckungs/Stech-Einheit) oder separat von Kammerabdeckung, einfacher wird.

Ferner ermöglicht die vorerwähnte verlagerbare Tiegelaufnahme, dass (bei entsprechender Ausgestaltung) durch das Stechen des Lochs in die Tiegelabdeckung des Probentiegels hervorgerufene mechanische Belastungen von mechanisch empfindlichen Komponenten der Vorrichtung, beispielsweise einer zur thermogravimetrischen Analyse (TG) vorgesehenen Waage, ferngehalten werden können.

Ein weiterer Vorteil der Ausführungsform mit verlagerbarer Tiegelaufnahme ergibt sich für den Fall, dass die Vorrichtung eine weitere Kammerabdeckung aufweist, die anstelle einer Abdeckungs/Stech-Einheit auf die Kammeröffnung der Probenkammer aufsetzbar ist. Nämlich kann in diesem Fall vorgesehen sein, dass der Probentiegel mitsamt bereits aufgestochener Tiegelabdeckung von der ersten Stellung (zum Abstellen des Probentiegels und Aufstechen der Tiegelabdeckung) in eine zweite Stellung verlagert wird, die bezüglich der ersten Stellung "weiter innen" (z. B. nach unten in die Vorrichtung versenkt) oder zumindest weiter weg von der Kammeröffnung vorgesehen ist, bevor dann (in der zweiten Stellung der Tiegelaufnahme) die Abdeckungs/Stech-Einheit von der Kammeröffnung der Probenkammer abgenommen und durch die andere Kammerabdeckung ersetzt wird.

Da sich der Probentiegel in der zweiten Stellung weiter innen bzw. zumindest weiter entfernt von der Kammeröffnung befindet, an der nach dem Abnehmen der Abdeckungs/Stech-Einheit ein gewisser Austausch zwischen Gasatmosphäre und Umgebungsluft stattfinden kann, ist die Probe während dieser Phase (Ersatz der Kammerabdeckung) besonders gut vor einem Kontakt mit Umgebungsluft geschützt.

In diesem Zusammenhang sei auch nochmals darauf hingewiesen, dass die weiter oben erwähnte Schutzgasdurchströmung der Probenkammer vom Inneren der Vorrichtung zur Kammeröffnung der Probenkammer hin vorteilhaft bereits einen Schutz der Probe vor einem Kontakt mit Umgebungsluft bewirkt. Dieser Schutz kann jedoch mittels der Verlagerung des Probentiegels (mit der bereits aufgestochenen Tiegelabdeckung) von der ersten Stellung in die zweite Stellung und somit weiter weg von der Kammeröffnung noch vorteilhaft gesteigert werden.

Gemäß eines weiteren Aspekts der Erfindung wird die eingangs gestellte Aufgabe durch ein Verfahren für eine thermische Analyse einer Probe mit den Merkmalen des Anspruchs 8 gelöst.

Die für die erfindungsgemäße Vorrichtung hier beschriebenen Ausführungsformen und besonderen Ausgestaltungen können, einzeln oder in beliebiger Kombination, in analoger Weise auch als Ausführungsformen bzw. besondere Ausgestaltungen des erfindungsgemäßen Verfahrens vorgesehen sein, und umgekehrt.

In einer Ausführungsform ist vorgesehen, dass die Kammerabdeckung und die Stecheinrichtung als eine Abdeckungs/Stech-Einheit baulich zusammengefasst ausgebildet sind, wobei nach dem Stechen des Lochs in die Tiegelabdeckung und vor der Durchführung der thermischen Analyse der Probe die Abdeckungs/Stech-Einheit von der Kammeröffnung der Probenkammer abgenommen und durch eine andere Kammerabdeckung ersetzt wird.

In einer Weiterbildung dieser Ausführungsform ist vorgesehen, dass die Erzeugung der definierten Gasatmosphäre durch eine mittels der Gasführungseinrichtung bewirkte Gasdurchströmung der Probenkammer realisiert wird, deren Strömungsrate während des Ersetzens der Abdeckungs/Stech-Einheit durch die andere Kammerabdeckung höher ist als während der Durchführung der thermischen Analyse.

In einer Ausführungsform des Verfahrens ist vorgesehen, dass in der Probenkammer eine zwischen einer ersten Stellung und einer zweiten Stellung verlagerbare Tiegelaufnahme ausgebildet ist, und dass
- das Einbringen des Probentiegels in die Probenkammer durch ein Abstellen des Probentiegels auf eine Abstellfläche der Tiegelaufnahme bei in der ersten Stellung befindlicher Tiegelaufnahme erfolgt,
- nach dem Aufsetzen der Kammerabdeckung auf die Kammeröffnung der Probenkammer und dem Erzeugen der Gasatmosphäre in der Probenkammer das Stechen des Lochs in die Tiegelabdeckung bei in der ersten Stellung befindlicher Tiegelaufnahme erfolgt,
- nach dem Stechen des Lochs in die Tiegelabdeckung und vor der Durchführung der thermischen Analyse der Probe eine Verlagerung der Tiegelaufnahme von der ersten Stellung in die zweite Stellung erfolgt.

Die zweite Stellung der verlagerbaren Tiegelaufnahme ist hierbei bevorzugt weiter weg von der Kammeröffnung und/oder weiter innen in der Vorrichtung vorgesehen als die erste Stellung. Insbesondere kann die Verlagerung der Tiegelaufnahme in Vertikalrichtung erfolgen, wobei die zweite Stellung weiter weg von der Kammeröffnung und/oder weiter innen in der Vorrichtung (z. B. weiter unten) als die erste Stellung vorgesehen ist.

In einer Weiterbildung ist vorgesehen, dass die Vorrichtung eine weitere Kammerabdeckung aufweist, die anstelle einer Abdeckungs/Stech-Einheit auf die Kammeröffnung der Probenkammer aufsetzbar ist, und dass das Abnehmen der Abdeckungs/Stech-Einheit von der Kammeröffnung und das Ersetzen derselben durch die andere Kammerabdeckung bei in der zweiten Stellung befindlicher Tiegelaufnahme erfolgt.

Ein Probentiegel mit Tiegelabdeckung, der in besonderer Weise für eine Verwendung für eine Vorrichtung und/oder in einem Verfahren der hier beschriebenen Art ausgebildet ist, kann z.B. aufweisen:
- einen wenigstens annähernd zylindrisch formgestalteten Probentiegel mit einem Innenraum zur Aufnahme der Probe und mit einer Tiegelöffnung,
- eine an der Tiegelöffnung des Probentiegels auf den Probentiegel aufsetzbare oder aufgesetzte Tiegelabdeckung,
wobei die Tiegelabdeckung aufweist:
- einen mit einem Öffnungsrand des Probentiegels verbindbaren oder verbundenen Deckel, insbesondere Schraubdeckel, mit einem darin ausgebildeten Deckelloch,
- eine den Innenraum des Probentiegels abdichtende, an der Innenseite des Deckels angeordnete, durchstechbare Dichtungslage.

Vorteilhaft kann bei einem derartigen Probentiegel mit Tiegelabdeckung das beim erfindungsgemäßen Verfahren vorgesehene Stechen eines Lochs in die Tiegelabdeckung so erfolgen, dass die hierfür eingesetzte Nadel durch das Deckelloch hindurchtritt und sodann die darunter befindliche Dichtungslage durchsticht.

Die Querschnitte einerseits des Deckellochs und andererseits der Nadel sind dementsprechend in Anpassung zueinander so zu wählen, dass die Nadel bzw. zumindest eine Spitze der Nadel durch das Deckelloch hindurch passt. In einer Ausführungsform ist eine Öffnungsfläche des Deckellochs um mindestens einen Faktor 2, insbesondere um einen Faktor 4, größer als eine maximale Querschnittsfläche eines beim Stechvorgang im Bereich des Deckellochs befindlichen Abschnitts der Nadel bzw. Nadelspitze. Andererseits ist es zumeist günstig, wenn diese Öffnungsfläche des Deckellochs um höchstens einen Faktor 10 größer als diese maximale Querschnittsfläche der Nadel(spitze) ist.

Ein Material und eine Dicke der Dichtungslage einerseits und ein Material und eine Formgestaltung die Nadel andererseits sind in Anpassung zueinander so zu wählen, dass mit der Nadel das Material der Dichtungslage durchdrungen und somit ein Loch in die Dichtungslage gestochen werden kann.

Vorteilhaft unabhängig von der Gestaltung der Nadel können Material und Formgestaltung (insbesondere z. B. Dicke) des Deckels je nach gewünschter mechanischer Stabilität des Deckels gewählt werden, da die Nadel beim Einstechvorgang den Deckel im Bereich des beispielsweise als eine geeignet bemessene Bohrung ausgebildeten Deckellochs durch den Deckel hindurchtritt.

Der Probentiegel kann einen Tiegelkorpus aufweisen, der zumindest annähernd die Form einer Schale oder eines Topfes besitzt.

In einer Ausführungsform ist der Probentiegel aus einem Metall oder einer Metalllegierung gebildet, wie z. B. Stahl, oder z. B. Wolfram, Titan oder Aluminium (oder einer Legierung davon), wobei den Innenraum des Probentiegels begrenzende Flächen z. B. beschichtet sein können. Als Material für eine solche Beschichtung kommen insbesondere Edelmetalle oder Edelmetalllegierungen in Betracht, wie z. B. Gold bzw. eine Goldlegierung.

In einer Ausführungsform ist vorgesehen, dass der Probentiegel einen kreisrunden Boden mit einem sich daran in Verwendungssituation nach oben hin anschließenden zylindrischen oder konischen Mantel aufweist. Der Boden und der Mantel können hierbei z. B. einstückig miteinander verbunden ausgebildet sein. Die Tiegelöffnung des Probentiegels wird in diesem Fall durch einen oberen Rand des Mantels definiert.

Der Probentiegel kann z. B. eine maximale laterale Ausdehnung im Bereich von 5 bis 15 mm und/oder eine Höhe im Bereich von 5 bis 15 mm besitzen, bevorzugt in einem Lateralausdehnung/Höhe-Verhältnis im Bereich von 1,0 bis 1,5. Eine Wandstärke des Tiegelkorpus kann z. B. im Bereich von 0,5 bis 1 mm liegen.

Die an der Tiegelöffnung des Probentiegels auf den Probentiegel aufsetzbare bzw. aufgesetzte Tiegelabdeckung kann wie bereits erwähnt zumindest einen Deckel und darunter (zum Inneren des Probentiegels hin) eine durchstechbare Dichtungslage aufweisen. In einer Ausführungsform ist der Deckel aus einem Metallmaterial (Metall oder Metalllegierung) wie z. B. Stahl, Titan oder Aluminium gebildet (z. B. aus dem gleichen Material wie der Probentiegel).

In einer Ausführungsform ist vorgesehen, dass der Deckel als ein Schraubdeckel mit einem Gewinde (Innen- oder Außengewinde) ausgebildet ist, das mit einem korrespondierenden Gewinde (Außen- bzw. Innengewinde) des Probentiegels im Bereich der Tiegelöffnung verschraubbar ist.

In einer Ausführungsform ist die den Innenraum des Probentiegels abdichtende, an der Innenseite des Deckels angeordnete, durchstechbare Dichtungslage als eine Folie aus einem Metallmaterial gebildet. Als Material für die Dichtungslage kommt vorteilhaft z. B. insbesondere Gold oder eine Goldlegierung in Betracht. Eine Dicke der Dichtungslage kann z. B. im Bereich von 50 bis 200 µm liegen.

Zur Realisierung der abdichtenden Wirkung kann z. B. vorgesehen sein, dass ein Rand der Dichtungslage umlaufend von dem Deckel auf einen oberen Randbereich des Probentiegels gedrückt wird, mit anderen Worten zwischen Deckel und Probentiegel eingeklemmt ist.

In einer vorteilhaften Weiterbildung ist vorgesehen, dass die Tiegelabdeckung ferner eine an der Innenseite der Dichtungslage angeordnete Stabilisierungslage mit einem darin koaxial zum Deckelloch ausgebildeten Stabilisierungslagenloch aufweist.

In diesem Fall kann z. B. vorgesehen sein, dass ein Rand des zweilagigen Konstrukts aus Dichtungslage und darunter angeordneter Stabilisierungslage umlaufend zwischen Deckel und Probentiegel eingeklemmt wird, um die Abdichtung des Innenraums des Probentiegels zu realisieren.

Bei dieser Weiterbildung ist mit der Stabilisierungslage vorteilhaft eine mechanische Abstützung der Dichtungslage geschaffen, so dass beim Stechen des Lochs in die Tiegelabdeckung (Durchstechen der Dichtungslage) die Dichtungslage bei Belastung mit der Nadel nicht zurückweichen kann und somit eine besser definierte und reproduzierbare Geometrie des eingestochenen Lochs erzielt wird.

Durch die koaxial zum Deckelloch und somit beim Einstechvorgang koaxial zur Nadel vorgesehene Anordnung des Stabilisierungslagenlochs in der Stabilisierungslage wird vorteilhaft vermieden, dass die Einstechbewegung der Nadel durch die Stabilisierungslage behindert wird.

In einer Ausführungsform ist eine Öffnungsfläche des Stabilisierungslagenlochs um mindestens einen Faktor 2, insbesondere um einen Faktor 4, größer als eine maximale Querschnittsfläche eines beim Stechvorgang im Bereich des Stabilisierungslagenlochs befindlichen Abschnitts der Nadel bzw. Nadelspitze.

In einer Ausführungsform der erfindungsgemäßen Vorrichtung umfasst diese Vorrichtung wenigstes einen Probentiegel samt Tiegelabdeckung der hier beschriebenen Art.

Eine Abdeckungs/Stech-Einheit für eine Vorrichtung für eine thermische Analyse einer Probe kann z.B. aufweisen: eine zur Abdeckung der Kammeröffnung vorgesehene Wandung; ein an der Wandung ausgebildetes Führungslager; und eine mittels des Führungslagers bewegbar gelagerte Nadel, die sich von einer bei aufgesetzter Abdeckungs/Stech-Einheit der Probenkammer zugewandten Innenseite der Wandung durch die Wandung hindurch bis auf eine bei aufgesetzter Abdeckungs/Stech-Einheit der Probenkammer abgewandten Außenseite der Wandung erstreckt.

Bei einer derartigen Abdeckungs/Stech-Einheit können insbesondere besondere Ausführungsformen und Ausgestaltungen vorgesehen sein, wie diese bereits im Zusammenhang mit der erfindungsgemäßen Vorrichtung hier beschriebenen sind.

Gemäß eines weiteren Aspekts der Erfindung wird eine Verwendung eines Verfahrens der hier beschriebenen Art zur Durchführung einer oder mehrerer thermischer Analysen einer Probe vorgeschlagen, ausgewählt aus der Gruppe bestehend aus Thermogravimetrische Analyse (TG), Differentialthermoanalyse (DTA) und Dynamische Differenzkalorimetrie (DSC).

In einer Ausführungsform dieser Verwendung ist eine Simultane Thermische Analyse umfassend eine der vorgenannten Analysemethoden und wenigstens eine weitere Analysemethode vorgesehen.

In einer vorteilhaften spezielleren Ausführungsform ist die Verwendung für eine thermische Analyse umfassend eine Ermittlung der Masse einer Probe im Verlauf eines Temperaturprogramms mittels Thermogravimetrie (TG) und eine simultane Ermittlung der Art und Menge an flüchtigen Zersetzungsprodukten der Probe im Verlauf dieses Temperaturprogramms mittels Massenspektroskopie (MS bzw. GC-MS) vorgesehen.

In einer Ausführungsform definiert das Temperaturprogramm eine Temperatur im Inneren der Probenkammer (Kammertemperatur), wofür das Verfahren z. B. ein Messen der Kammertemperatur und darauf basierend eine Ansteuern der Temperiereinrichtung umfassen kann, bevorzugt mit einer Regelung (z. B. PID-Regelung) der Kammertemperatur.

Abweichend davon kann das Temperaturprogramm alternativ auch einen vorbestimmten zeitlichen Verlauf der Probentemperatur definieren, wofür dementsprechend z. B. basierend auf der gemessenen Probentemperatur die entsprechende Ansteuerung (insbesondere Regelung) der Temperiereinrichtung erfolgen kann.

Je nach konkreter Ausführung des Verfahrens bzw. einer hierbei eingesetzten Messeinrichtung werden eine oder mehrere weitere Messgrößen der Probe, insbesondere z. B. Messgrößen betreffend Eigenschaften der Probe gemessen. Beispielsweise bei Einsatz der Methode der Thermogravimetrie (TG) wird als weitere Messgröße das Gewicht bzw. die Masse der Probe im Verlauf einer Temperaturveränderung bzw. eines Temperaturprogramms gemessen. Alternativ oder zusätzlich kann eine weitere Messgröße sich z. B. auf Art und/oder Menge an flüchtigen Produkten beziehen (z. B. ermittelt mit Hilfe von Gaschromatographie und/oder Massenspektroskopie), und/oder wenigstens eine weitere Temperatur und/oder Temperaturdifferenz umfassen (z. B. bei DSC). Bevorzugt umfasst das Verfahren ein Aufzeichnen von Messdaten im Verlauf des Temperaturprogramms, insbesondere von Daten, die den temperaturabhängigen und/oder zeitabhängigen Verlauf wenigstens einer Temperatur von Kammertemperatur und Probentemperatur (bevorzugt beides) repräsentieren. Durch Auswertung derartiger Daten, während der Temperierung und/oder nach Abschluss des Temperaturprogramms, können z. B. eine oder mehrere interessierende Materialparameter der dem Verfahren unterzogenen Probe ermittelt werden.

Die Erfindung wird nachfolgend anhand von Ausführungsbeispielen mit Bezug auf die beigefügten Zeichnungen weiter beschrieben. Es stellen dar:
- Fig. 1: eine Schnittansicht einer Vorrichtung für eine thermische Analyse einer Probe gemäß eines Ausführungsbeispiels,
- Fig. 2: ein in der Darstellung etwas vereinfachtes Detail der Schnittansicht von Fig. 1, nämlich eine Abdeckungs/Stech-Einheit der Vorrichtung von Fig. 1,
- Fig. 3: eine geschnittene perspektivische Ansicht der Abdeckungs/Stech-Einheit von Fig. 2,
- Fig. 4: eine perspektivische Ansicht eines Haubenteils der Abdeckungs/Stech-Einheit der Fig. 2 und 3,
- Fig. 5: eine schematische Explosionsansicht eines Probentiegels mit Tiegelabdeckung gemäß eines Ausführungsbeispiels,
- Fig. 6: eine Draufsicht eines Deckels der Tiegelabdeckung von Fig. 5,
- Fig. 7: eine Draufsicht einer Stabilisierungslage der Tiegelabdeckung von Fig. 5, und
- Fig. 8: eine Schnittansicht einer Vorrichtung für eine thermische Analyse einer Probe gemäß eines weiteren Ausführungsbeispiels.

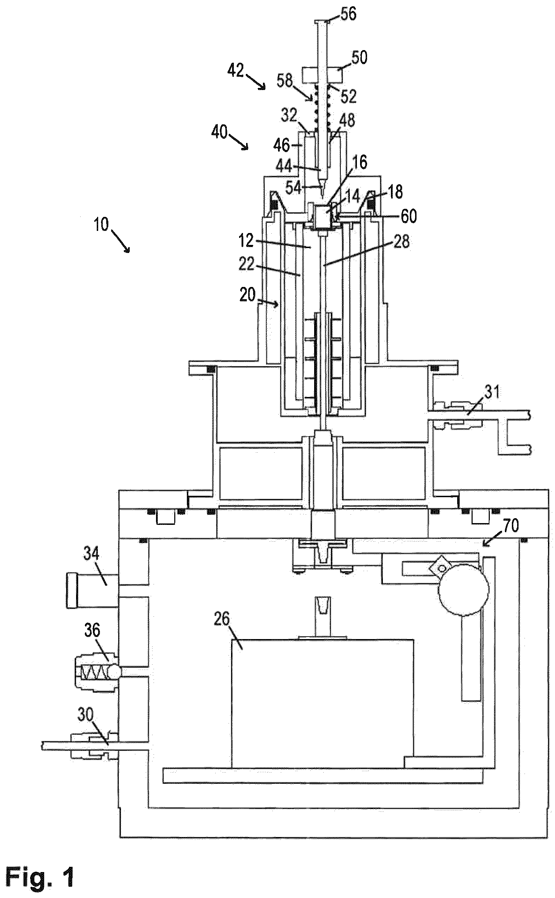

Fig. 1 zeigt ein Ausführungsbeispiel einer Vorrichtung 10 für eine thermische Analyse einer Probe. Die Vorrichtung 10 weist eine Probenkammer 12 zur Aufnahme eines Probentiegels 14 mit einer darauf aufgesetzten Tiegelabdeckung 16 auf. Bei der thermischen Analyse befindet sich die zu analysierende Probe im Inneren des Probentiegels 14.

Die Probenkammer 12 besitzt in einem oberen Bereich der Vorrichtung 10 eine Kammeröffnung 18, durch welche hindurch zur Vorbereitung der Analyse der Probentiegel 14 in die Probenkammer 12 eingebracht werden kann.

Die Vorrichtung 10 weist ferner eine Temperiereinrichtung 20 auf, mittels welcher bei der Analyse der Probe die Probenkammer 12 und somit die im Probentiegel 14 befindliche Probe temperiert wird. Die Temperiereinrichtung 20 weist im dargestellten Beispiel einen hohlzylindrischen Heizmantel 22 (z. B. elektrische Widerstandsheizung) auf.

Im dargestellten Ausführungsbeispiel ist die Vorrichtung 10 dazu ausgebildet, ein Verfahren zur thermischen Analyse der Probe durchzuführen, bei welchem die Probe gemäß eines vorbestimmten Temperaturprogramms temperiert wird, in dessen Verlauf eine Kammertemperatur im Inneren der Probenkammer 12 verändert wird, wobei im Verlauf des Temperaturprogramms mittels einer Messeinrichtung der Vorrichtung 10 eine Probentemperatur und zusätzlich eine oder mehrere weitere Messgrößen betreffend Eigenschaften der Probe gemessen werden.

Im dargestellten Ausführungsbeispiel ist bei der thermischen Analyse der Probe eine thermogravimetrische Analyse (TG) vorgesehen, bei welcher (als weitere Messgröße) die Veränderung der Masse der Probe im Verlauf des Temperaturprogramms gemessen wird.

Die Messeinrichtung umfasst daher im dargestellten Ausführungsbeispiel neben einer Temperaturmesseinrichtung zur Messung der Probentemperatur insbesondere eine Waage 26, mittels welcher im Verlauf des Temperaturprogramms das Gewicht der Probe mitsamt der zur Aufnahme und Lagerung der Probe auf der Waage 26 vorgesehenen Komponenten gemessen wird. Im Beispiel umfassen diese Komponenten z. B. den Probentiegel 14 samt Tiegelabdeckung 16 und einen Probentiegelträger 28. Durch eine Auswertung des im Verlauf des Temperaturprogramms mittels der Waage 26 gemessenen Gewichts wird die temperaturabhängige Veränderung der Masse der Probe erhalten.

Zum Erzeugen einer definierten Gasatmosphäre bzw. Gasdurchströmung in der Probenkammer 12 weist die Vorrichtung 10 eine Gasführungseinrichtung auf, welche im dargestellten Ausführungsbeispiel insbesondere einen in einem unteren Bereich der Probenkammer 12 angeordneten Gaseinlass 30 aufweist. Im dargestellten Beispiel ist im unteren Bereich der Probenkammer 12 ein weiterer Gaseinlass 31 angeordnet. Über diese Gaseinlässe 30, 31 kann ein Gas (z. B. Stickstoff, Argon oder Helium, oder ein Gemisch enthaltend wenigstens ein derartiges Inertgas) in diesen Bereich der Probenkammer 12 einströmen gelassen werden.

Im dargestellten Beispiel und in der in Fig. 1 dargestellten Situation weist die Gasführungseinrichtung ferner einen in einem oberen Bereich der Probenkammer 12 angeordneten Gasauslass 32 auf, über welchen das Schutzgas in diesem Bereich der Probenkammer 12 ausströmen gelassen werden kann. Im Beispiel umfasst die Gasführungseinrichtung der Vorrichtung 10 des Weiteren einen Drucksensor 34 zur Messung eines Drucks in der Probenkammer 12 sowie ein Überdruck-Auslassventil (Sicherheitsventil) 36, welches im Falle eines übermäßigen Drucks Gas aus der Probenkammer 12 auslässt.

Um die Probenkammer 12 der Vorrichtung 10 bei der Durchführung der thermischen Analyse nach oben hin gasdicht gegenüber der Atmosphäre (Umgebungsluft) abzudecken, weist die Vorrichtung 10 ferner eine oder mehrere (wahlweise) auf die Kammeröffnung 18 der Probenkammer 12 aufsetzbare Kammerabdeckungen auf. In der in Fig. 1 dargestellten Situation ist eine solche Kammerabdeckung 40 auf die Kammeröffnung 18 aufgesetzt.

Eine Besonderheit der Vorrichtung 10 besteht darin, dass zur Verringerung der Gefahr einer unerwünschten Veränderung der Probe infolge chemischer oder physikalischer Reaktionen mit Bestandteilen der Umgebungsluft, der Probentiegel 14 in mittels der Tiegelabdeckung 16 luftdicht verschlossenem Zustand in die Probenkammer 12 eingebracht und dort unter mittels der Gasführungseinrichtung erzeugter Gasatmosphäre geöffnet werden kann.

Zu diesem Zweck weist die Vorrichtung 10 ferner eine Stecheinrichtung 42 auf, welche mit einer Nadel 44 (z. B. aus Stahl oder einer anderen Metalllegierung) ausgestattet dazu ausgebildet ist, bei in der Probenkammer 12 aufgenommenem Probentiegel 14 und auf der Kammeröffnung 18 aufgesetzter Kammerabdeckung 40 mit der Nadel 44 ein Loch in die Tiegelabdeckung 16 des Probentiegels 14 zu stechen.

Im dargestellten Beispiel sind die Kammerabdeckung 40 und die Stecheinrichtung 42 als eine Abdeckungs/Stech-Einheit 40, 42 baulich zusammengefasst ausgebildet und in Fig. 1 ist eine Situation dargestellt, in der nach dem Einbringen des Probentiegels 14 durch die Kammeröffnung 18 hindurch in die Probenkammer 12 die Abdeckungs/Stech-Einheit 40, 42 auf die Kammeröffnung 18 aufgesetzt wurde.

In dieser Situation kann mit der Nadel 44 das besagte Loch in die Tiegelabdeckung 16 des Probentiegels 14 gestochen werden.

Die Abdeckungs/Stech-Einheit 40, 42 weist im dargestellten Ausführungsbeispiel eine zur Abdeckung der Kammeröffnung 18 dienende Wandung 46 und ein an der Wandung 46 ausgebildetes Führungslager 48 (Gleitlager) zur bewegbaren Lagerung der Nadel 44 auf. Die Nadel 44 erstreckt sich hierbei von einer bei aufgesetzter Abdeckungs/Stech-Einheit 40, 42 der Probenkammer 12 zugewandten Innenseite der Wandung 46 durch die Wandung 46 hindurch bis auf eine bei aufgesetzter Abdeckungs/Stech-Einheit 40, 42 der Probenkammer 12 abgewandten Außenseite der Wandung 46.

Im dargestellten Beispiel wird ein für den Stechvorgang vorgesehener "Antrieb der Nadel" durch eine mechanische Krafteinwirkung auf den auf der Außenseite der Wandung 46 befindlichen Abschnitt der Nadel 44 bewerkstelligt.

Zu diesem Zweck weist die Stecheinrichtung 42 ein vertikalbeweglich geführtes Gewicht 50 auf, das durch eine manuelle Bedienhandlung eines Benutzers aus einer vorbestimmten Höhe bezüglich der Nadel 44 auf einen am Schaft der Nadel 44 ausgebildeten radialen Vorsprung 52 der Nadel 44 fallen gelassen werden kann, um somit die Nadel 44 in Fig. 1 nach unten anzutreiben und mittels einer Nadelspitze 54 der Nadel 44 das besagte Loch in die Tiegelabdeckung 16 einstechen zu lassen.

Zur Vorbestimmung der Höhe, aus welcher das Gewicht 50 auf den radialen Vorsprung 52 der Nadel 44 fallen gelassen wird, ist im dargestellten Beispiel z. B. ein weiterer radialer Vorsprung 56 am Schaft der Nadel 44 ausgebildet. Bei der vorerwähnten manuellen Bedienhandlung kann der Benutzer das am Schaft der Nadel 44 geführte Gewicht 50 zunächst anheben, bis dieses den hierbei als Anschlag dienenden weiteren radialen Vorsprung 56 erreicht, und dann das Gewicht 50 loslassen. Alternativ oder zusätzlich zur Ausbildung eines die Fallhöhe definierenden Anschlags (Vorsprung 56) könnten z. B. auch eine oder mehrere Markierungen ("Skala") am Schaft der Nadel 44 vorgesehen sein, um somit ein Anheben des Gewichts 50 auf eine oder mehrere somit definierte Fallhöhen zu ermöglichen.

Die Stecheinrichtung 42 weist im dargestellten Beispiel ferner eine Federeinrichtung 58 auf, die zur Vorbelastung der Nadel 44 entgegen ihrer Stechrichtung dient und im dargestellten Beispiel als eine den Schaft der Nadel 44 umgebende Spiraldruckfeder ausgebildet ist, welche sich einerseits an der Wandung 46 und andererseits an dem radialen Vorsprung 52 der Nadel 44 abstützt. Vorteilhaft kann damit der Einstechvorgang der Nadel 44 mit einer wohldefiniert reproduzierbaren Einstechtiefe erfolgen, wobei das Ausmaß dieser Einstechtiefe letztlich abhängt von der Charakteristik der Federeinrichtung 58, der Fallhöhe und der Masse des Gewichts 50, und Eigenschaften der Nadelspitze (Material, Geometrie) in Verbindung mit Eigenschaften der davon zu durchstechenden Tiegelabdeckung 16 (z. B. Material und Dicke einer zu durchstechenden Dichtungslage).

Abweichend vom dargestellten Ausführungsbeispiel könnte die Stecheinrichtung 42 z. B. auch einen mechanischen Anschlag zur Begrenzung der Einstechtiefe der Nadel 44 aufweisen.

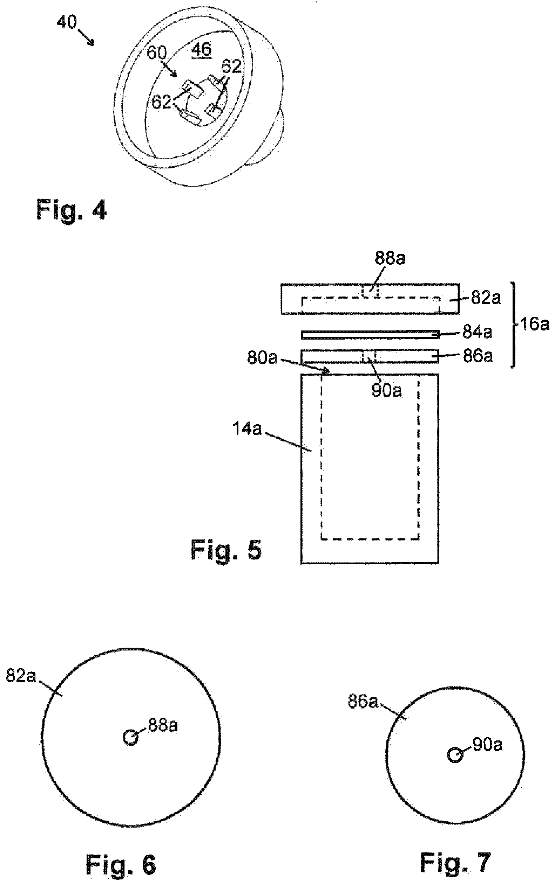

Als im Rahmen der Erfindung zumeist vorteilhaft hat es sich erwiesen, wenn die Kammerabdeckung 40 mit deren Wandung 46 ein (domartiges) "Haubenteil" (vgl. z. B. Fig. 4) ausbildet, so dass durch das Aufsetzen der Kammerabdeckung 40 bzw. im dargestellten Beispiel der Abdeckungs/Stech-Einheit 40, 42 die Probenkammer 12 bzw. im Bereich der Kammeröffnung 18 domartig erweitert wird. In diesem Fall ist ein Gasauslass der Kammerabdeckung 40 bevorzugt in einem distalen Bereich der Haubenform angeordnet (wie für den im dargestellten Beispiel vorgesehenen Gasauslass 32 der Fall).

Die Stecheinrichtung 42 weist im dargestellten Beispiel ferner eine mechanische Zentriereinrichtung 60 ("Zentrierhilfe") auf, mittels welcher vor der Durchführung des Einstechvorganges der Probentiegel 14 samt Tiegelabdeckung 16 bezüglich der Stecheinrichtung 42 und somit der Nadel 44 zentriert wird. Vorteilhaft erfolgt das Einstechen der Tiegelabdeckung 16 damit an einer wohldefinierten Stelle der Tiegelabdeckung 16.

Die Zentriereinrichtung 60 der Abdeckungs/Stech-Einheit 40, 42 wird im dargestellten Beispiel durch mehrere Zentrierbacken 62 gebildet, welche derart an der Innenseite der Wandung 46 angeordnet und z. B. einstückig mit der Wandung 46 ausgebildet sind, dass diese bei der Durchführung des Einstechvorganges eine Mantelfläche des Probentiegels 14 und/oder eine Mantelfläche der Tiegelabdeckung 16 kontaktieren, um damit den Probentiegel 14 samt Tiegelabdeckung 16 in eine bestimmte Position bezüglich der Nadel 44 zu zwingen (Zentrierung), bevor mittels der Nadel 44 das Loch in die Tiegelabdeckung 16 des Probentiegels 14 gestochen wird.

Darüber hinaus besitzt die verlagerbare Tiegelaufnahme im dargestellten Beispiel den Vorteil, dass der Probentiegel 14 mitsamt bereits aufgestochener Tiegelabdeckung 16 von der ersten Stellung in die zweite Stellung verlagert werden kann, bevor dann in der zweiten Stellung die Abdeckungs/Stech-Einheit 40, 42 von der Kammeröffnung 18 der Probenkammer 12 abge-nommen und durch die andere Kammerabdeckung ersetzt wird. Da sich der Probentiegel 14 in der zweiten Stellung weiter unten und somit weiter entfernt von der Kammeröffnung 18 befindet, an der nach dem Abnehmen der Abdeckungs/Stech-Einheit 40, 42 in der Praxis ein gewisser Gasaustausch zwischen Gasatmosphäre und Umgebungsluft unvermeidlich ist, ist die Probe während dieser Probenvorbereitungsphase (Ersatz der Kammerabdeckung) noch besser vor einem Kontakt mit Umgebungsluft geschützt.

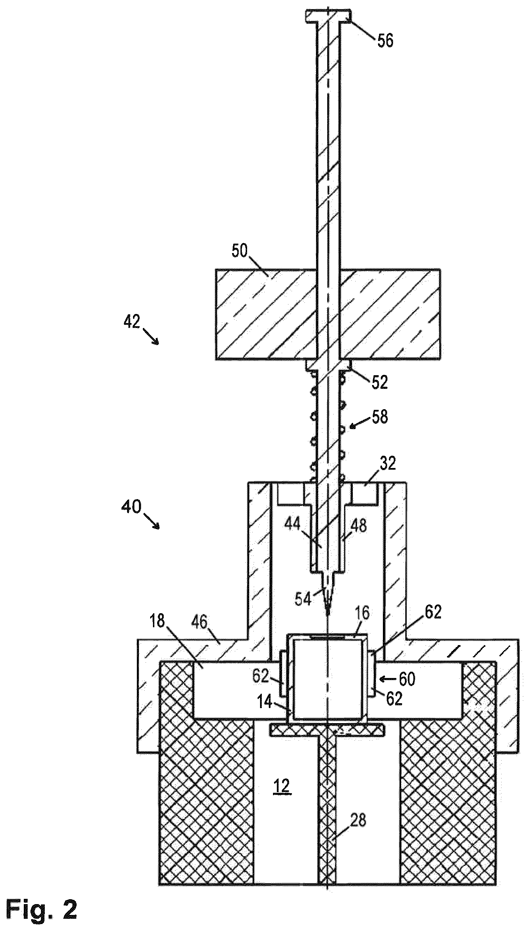

Fig. 2 zeigt vergrößert und etwas schematisiert vereinfacht die Abdeckungs/Stech-Einheit 40, 42 aus der Schnittansicht von Fig. 1.

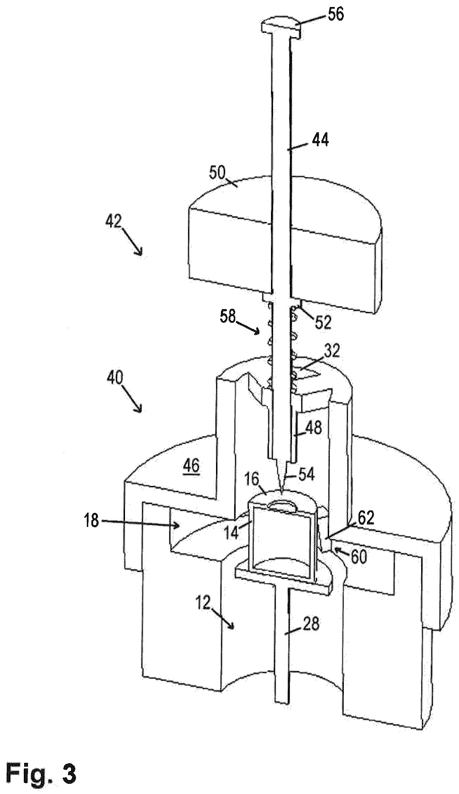

Fig. 3 zeigt eine geschnittene perspektivische Ansicht der Abdeckungs/Stech-Einheit 40, 42.

Fig. 4 zeigt eine perspektivische Ansicht eines "Haubenteils" der Abdeckungs/Stech-Einheit 40, 42 der Fig. 2 und 3, umfassend die Wandung 46 und die von mehreren Zentrierbacken 62 gebildete Zentriereinrichtung 60.

Falls die Kammerabdeckung bei der Durchführung der thermischen Analyse abgesehen von der Funktion zum Schutz eines Austausches von Medien und/oder Energie (Wärme) zwischen Probenkammer 12 und Umgebung (in Fig. 1 überhalb der Abdeckungs/Stech-Einheit 40, 42) noch weitere Funktion besitzt, ist es vorteilhaft, wenn nach dem Stechen des Lochs in die Tiegelabdeckung 16 und vor der Durchführung der thermischen Analyse der Probe die Abdeckungs/Stech-Einheit 40, 42 durch eine hinsichtlich der genannten weiteren Funktion(en) optimierte Kammerabdeckung ersetzt wird. Eine solche weitere Funktion kann es z. B. sein, einen Trägergasauslass bereitzustellen, durch welchen hindurch bei der thermischen Analyse der Probe als Trägergas fungierendes Gas mitsamt von der Probe stammenden gasförmigen Komponenten aus der Probenkammer 12 heraus zu einer Analyseeinrichtung (z. B. umfassend ein Massenspektrometer) weitergeleitet wird.

Im dargestellten Ausführungsbeispiel weist die Vorrichtung 10 daher eine (nicht dargestellte) weitere Kammerabdeckung auf, die anstelle der Abdeckungs/Stech-Einheit 40, 42 auf die Kammeröffnung 18 der Probenkammer 12 aufgesetzt werden kann und die ähnlich dem Gasauslass 32 der Abdeckungs/Stech-Einheit 40, 42 einen Gasauslass aufweist, an welchem eine zu einer Analyseeinrichtung führende Gasleitung anschließbar bzw. angeschlossen ist.

Ein unter Verwendung der Vorrichtung 10 durchgeführtes Verfahren für eine thermische Analyse der Probe kann insbesondere folgende Schritte umfassen:
- Einbringen des Probentiegels 14 samt darauf aufgesetzter Tiegelabdeckung 16 und darin aufgenommener Probe durch die Kammeröffnung 18 der Probenkammer 12 in die Probenkammer 12,
- Aufsetzen der Abdeckungs/Stech-Einheit 40, 42 auf die Kammeröffnung 18 der Probenkammer 12 und Erzeugen einer Gasatmosphäre in der Probenkammer 12 mittels der Gasführungseinrichtung 30, 31, 32,
- Stechen eines Lochs in die Tiegelabdeckung 16 des Probentiegels 12 mittels der Stecheinrichtung 42 der Abdeckungs/Stech-Einheit 40, 42,
- Durchführung der thermischen Analyse der Probe mittels der Temperiereinrichtung 20 zum Temperieren der Probenkammer 12 gemäß eines vorbestimmten Temperaturprogramms (zeitabhängiger Temperaturverlauf) und der Messeinrichtung zur Messung der Probentemperatur und der Massenveränderung der Probe.

Falls das Verfahren für die thermische Analyse der Probe hierbei simultan zur Thermogravimetrie (TG) eine zeitabhängige Ermittlung der Art und Menge an flüchtigen Zersetzungsprodukten der Probe im Verlauf des betreffenden Temperaturprogramms mittels Massenspektroskopie (MS) vorsieht, so wird die Abdeckungs/Stech-Einheit 40, 42 nach dem Stechen des Lochs in die Tiegelabdeckung 16, jedoch vor der Durchführung der thermischen Analyse der Probe von der Kammeröffnung 18 der Probenkammer 12 abgenommen und durch eine oben bereits erwähnte andere Kammerabdeckung ersetzt, an welcher ein Gasauslass ausgebildet ist, über welchen bei der thermischen Analyse die flüchtigen Zersetzungsprodukte der Probe zu einem Massenspektrometer geführt werden.

Hierbei wird mittels der Gasführungseinrichtung eine Gasdurchströmung der Probenkammer 12 bewirkt, indem das betreffende Gas, das im Hinblick auf dessen Funktion im Zusammenhang mit der Massenspektroskopie auch als Trägergas bezeichnet werden kann, mit einer vorbestimmten Strömungsrate im unteren Bereich der Probenkammer 12 am Gaseinlass 30 und/oder 31eingelassen und im oberen Bereich der Probenkammer 12 am Gasauslass der Kammerabdeckung (ggf. z. B. über eine Trennsäule einer Gaschromatographie-Einrichtung) zum Massenspektrometer hin ausgelassen.

In ganz ähnlicher Weise kann eine solche Gasdurchströmung der Probenkammer 12 bereits während der eigentlichen Analyse vorausgehenden Phasen der Probenvorbereitung vorgesehen sein, nämlich beim Einbringen des Probentiegels 14 in die Probenkammer 12 sowie beim Abnehmen der Abdeckungs/Stech-Einheit 40, 42 von der Kammeröffnung 18 und nachfolgendem Aufsetzen der anderen Kammerabdeckung auf die Kammeröffnung 18.

In diesem Zusammenhang ist es von Vorteil, dass die Abdeckungs/Stech-Einheit 40, 42 wie bereits mit Bezug auf Fig. 1 beschrieben den Gasauslass 32 aufweist, durch den hindurch bei auf der Kammeröffnung 18 aufgesetzter Abdeckungs/Stech-Einheit 40, 42 das über den Gaseinlass 30 der Vorrichtung 10 in die Probenkammer 12 einströmende Gas aus der Probenkammer 12 nach außen in die Umgebung abströmen kann. Damit kann nach dem Aufsetzen der Abdeckungs/Stech-Einheit 40, 42 vorteilhaft rasch Luft aus der Probenkammer 12 verdrängt und durch die Gasatmosphäre ("Schutzgasatmosphäre") ersetzt werden, in welcher sodann das Öffnen der Tiegelabdeckung 16 (durch Einstechen mit der Nadel 44) erfolgen kann.

Bevorzugt ist hierbei vorgesehen, dass die mittels der Gasführungseinrichtung bewirkte Gasdurchströmung der Probenkammer 12 so bewirkt wird, dass deren Strömungsrate während des Einbringens des Probentiegels 14 in die Probenkammer 12 und/oder während des Ersetzens der Abdeckungs/Stech-Einheit 40, 42 durch die andere Kammerabdeckung höher ist (z. B. um wenigstens einen Faktor 2) als während der Durchführung der thermischen Analyse.

Zurückkommend auf Fig. 1 ist bei dem darin dargestellten Ausführungsbeispiel der Vorrichtung 10 durch den Probentiegelträger 28 bzw. dessen in Fig. 1 oberes Ende eine Tiegelaufnahme mit einer Abstellfläche zum darauf Abstellen des Probentiegels 14 ausgebildet, wobei in diesem Beispiel eine Besonderheit darin besteht, dass diese Tiegelaufnahme in Vertikalrichtung verlagerbar ist zwischen einer in Fig. 1 gezeigten ersten Stellung zum Abstellen des Probentiegels 14 und zum Stechen des Lochs in die Tiegelabdeckung 16 und einer (nicht dargestellten) zweiten Stellung zum Durchführen der thermischen Analyse der Probe.

In dieser zweiten Stellung befindet sich der Probentiegel 14 zwecks Durchführung der thermischen Analyse etwa im Zentrum des Heizmantels 22 der Temperiereinrichtung 20. Die zweite Stellung der verlagerbaren Tiegelaufnahme bzw. des verlagerbaren Probentiegelträgers 28 ist demnach weiter unten als die in Fig. 1 gezeigte erste Stellung vorgesehen, d. h. weiter weg von der Kammeröffnung 18 und weiter innen in der Vorrichtung 10.

In der ersten Stellung ist das Einbringen und Abstellen des z. B. mit einer Pinzette gehaltenen Probentiegels 14 in die Vorrichtung 10 (durch die Kammeröffnung der Probenkammer 12 hindurch) vereinfacht, wohingegen in der zweiten Stellung eine präzise Temperierung der Probe während der thermischen Analyse gewährleistet werden kann.

Darüber hinaus besitzt die verlagerbare Tiegelaufnahme im dargestellten Beispiel den Vorteil, dass eine durch das Einstechen des Lochs in die Tiegelabdeckung 16 des Probentiegels 14 hervorgerufene mechanische Belastung sich nicht auf die in diesem Beispiel zur thermogravimetrischen Analyse (TG) vorgesehenen Waage 26 überträgt. Nämlich ist in der ersten Stellung der Probentiegelträger 28 mit Hilfe einer Hubeinrichtung 70 nach oben angehoben und damit mechanisch von der Waage 26 entkoppelt. Erst nach dem Stechen des Lochs in die Tiegelabdeckung 16, jedoch vor Durchführung der thermischen Analyse, wird der Probentiegelträger 28 mit Hilfe der Hubeinrichtung 70 nach unten abgesenkt und somit auf der Waage 26 abgestellt.

Darüber hinaus besitzt die verlagerbare Tiegelaufnahme im dargestellten Beispiel den Vorteil, dass der Probentiegel 14 mitsamt bereits aufgestochener Tiegelabdeckung 16 von der ersten Stellung in die zweite Stellung verlagert werden kann, bevor dann in der zweiten Stellung die Abdeckungs/Stech-Einheit 40, 42 von der Kammeröffnung 18 der Probenkammer 12 abgenommen und durch die andere Kammerabdeckung ersetzt wird. Da sich der Probentiegel 14 in der zweiten Stellung weiter unten und somit weiter entfernt von der Kammeröffnung 18 befindet, an der nach dem Abnehmen der Abdeckungs/Stech-Einheit 40, 42 in der Praxis ein gewisser Gasaustausch zwischen Gasatmosphäre und Umgebungsluft unvermeidlich ist, ist die Probe während dieser Probenvorbereitungsphase (Ersatz der Kammerabdeckung) noch besser vor einem Kontakt mit Umgebungsluft geschützt.

Da im dargestellten Ausführungsbeispiel die zwischen der ersten Stellung und der zweiten Stellung verlagerbare Tiegelaufnahme (Probentiegelträger 28) ausgebildet ist, kann das mit der Vorrichtung 10 durchgeführte Verfahren also vorteilhaft vorsehen,
- dass das Einbringen des Probentiegels 14 in die Probenkammer 12 durch das Abstellen des Probentiegels 14 auf eine am oberen Ende des Probentiegelträgers 28 vorgesehene Abstellfläche der Tiegelaufnahme bei in der ersten Stellung befindlicher Tiegelaufnahme erfolgt,
- dass nach dem Aufsetzen der Kammerabdeckung (im Beispiel: Abdeckungs/Stech-Einheit 40, 42) auf die Kammeröffnung 18 der Probenkammer 12 und dem Erzeugen einer definierten Gasatmosphäre (und bevorzugt Gasdurchströmung) in der Probenkammer 12 das Stechen des Lochs in die Tiegelabdeckung 16 bei in der ersten Stellung befindlicher Tiegelaufnahme erfolgt, und
- dass nach dem Stechen des Lochs in die Tiegelabdeckung 16 und vor der Durchführung der thermischen Analyse der Probe eine Verlagerung der Tiegelaufnahme von der ersten Stellung in die zweite Stellung erfolgt.

Bei der nachfolgenden Beschreibung von weiteren Ausführungsbeispielen werden für gleichwirkende Komponenten die gleichen Bezugszahlen verwendet, jeweils ergänzt durch einen kleinen Buchstaben zur Unterscheidung der Ausführungsform. Dabei wird im Wesentlichen nur auf die Unterschiede zu dem bzw. den bereits beschriebenen Ausführungsbeispielen eingegangen und im Übrigen hiermit ausdrücklich auf die Beschreibung vorangegangener Ausführungsbeispiele verwiesen.

Nachfolgend wird mit Bezug auf die Fig. 5 bis 7 ein Ausführungsbeispiel eines bei einem erfindungsgemäßen Verfahren besonders vorteilhaft einsetzbaren Probentiegels 14a mit einer Tiegelabdeckung 16a beschrieben.

Fig. 5 zeigt den Probentiegel 14a mit Tiegelabdeckung 16a in einer schematischen Expolsionsansicht von der Seite.

Der Probentiegel 14a besitzt eine zylindrische Form mit einem Innenraum zur Aufnahme der Probe und mit einer Tiegelöffnung 80a an einer oberen Stirnseite dieser zylindrischen Form.

Die an der Tiegelöffnung 80a des Probentiegels 14a auf den Probentiegel 14a aufsetzbare oder aufgesetzte Tiegelabdeckung 16a besteht im dargestellten Beispiel aus einem Deckel 82a, einer durchstechbaren Dichtungslage 84a (Dichtungsfolie) und eine Stabilisierungslage 86a.

Der Deckel 82a ist als ein Schraubdeckel ausgebildet und an seinem Umfangsbereich umlaufend mit einem Öffnungsrand des Probentiegels 14a verbindbar (hier: verschraubbar). Im Beispiel besitzt der Deckel 82a an seinem Umfangsrandbereich ein Innengewinde, das mit einem korrespondierenden Außengewinde des Probentiegels 14a verschraubbar ist. Außerdem besitzt der Deckel 82a ein mittig angeordnetes, den Deckel 82a durchsetzendes Deckelloch 88a.

Die durchstechbare Dichtungslage 84a ist im montierten Zustand der "Probentiegel/Tiegelabdeckung-Kombination" 14a, 16a an der Innenseite des Deckels 82a angeordnet und dient in diesem Zustand dazu, den Innenraum des Probentiegels 14a abzudichten.

Die Stabilisierungslage 86a ist im montierten Zustand an der Innenseite der durchstechbaren Dichtungslage 84a angeordnet. Außerdem besitzt die Stabilisierungslage 86a ein koaxial zum Deckelloch 88a angeordnetes, die Stabilisierungslage 86a durchsetzendes Stabilisierungslagenloch 90a.

Im montierten Zustand der Probentiegel/Tiegelabdeckung-Kombination14a, 16a wird ein Rand des zweilagigen Konstrukts aus Dichtungslage 84a und darunter angeordneter Stabilisierungslage 86a umlaufend zwischen dem Deckel 82a und dem Probentiegel 14a eingeklemmt.

Im dargestellten Beispiel ist der Probentiegel 14a einstückig (Boden und Mantel) aus Stahl gebildet, wobei den Innenraum begrenzende Flächen mit einer Goldlegierung beschichtet sind. Die Tiegelabdeckung 16a besteht im dargestellten Beispiel aus Stahl (Deckel 82a) und einer Goldlegierung (Dichtungslage 84a und Stabilisierungslage 86a).

Bei dem Probentiegel 14a mit Tiegelabdeckung 16a kann das Stechen eines Lochs in die Tiegelabdeckung 16a vorteilhaft so erfolgen, dass die hierfür eingesetzte Nadel (z. B. Nadel 44 in den Fig. 1 bis 4) durch das Deckelloch 88a hindurchtritt und sodann die darunter befindliche Dichtungslage 84a durchsticht. Mit der Stabilisierungslage 86a ist hierbei vorteilhaft eine mechanische Abstützung der Dichtungslage 84a geschaffen, so dass beim Stechen des Lochs in die Dichtungslage 84a diese bei der Belastung durch die Nadel nicht zurückweichen kann und somit eine wohldefinierte und reproduzierbare Geometrie des Lochs erzielt wird. Durch das Vorsehen des Stabilisierungslagenlochs 90a wird vorteilhaft vermieden, dass die Einstechbewegung der Nadel durch die Stabilisierungslage 86a behindert wird.

Fig. 6 zeigt eine Draufsicht des Deckels 82a der Tiegelabdeckung 16a.

Fig. 7 zeigt eine Draufsicht der Stabilisierungslage 86a der Tiegelabdeckung 16a.

Die Durchmesser einerseits des Deckellochs 88a und des Stabilisierungslagenlochs 90a und andererseits der Nadel sind in Anpassung zueinander so zu wählen, dass die Nadel bzw. zumindest eine Spitze der Nadel durch das Deckelloch 88a und bevorzugt auch durch das Stabilisierungslagenloch 90a hindurch passt. Im dargestellten Beispiel sind die Durchmesser des Deckellochs 88a und des Stabilisierungslagenloch 90a gleich groß bemessen und betragen jeweils ca. 0,5 mm.

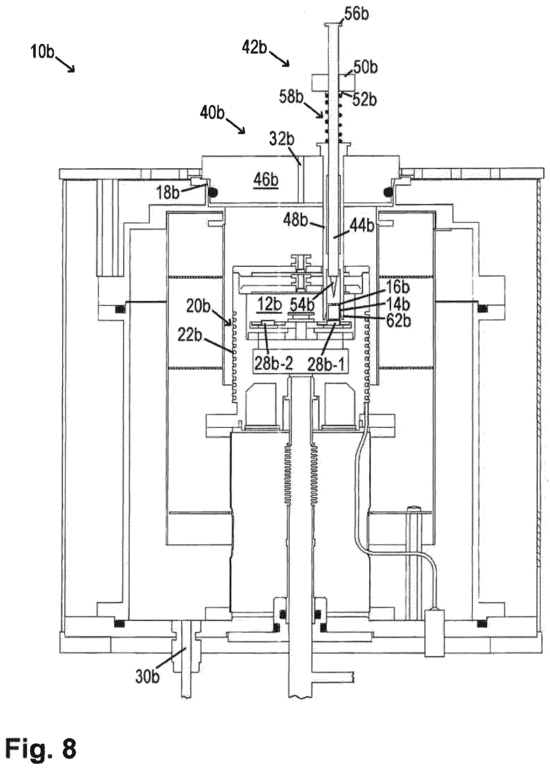

Fig. 8 zeigt ein weiteres Ausführungsbeispiel einer Vorrichtung 10b für eine thermische Analyse einer Probe. Wie die bereits mit Bezug auf Fig. 1 beschriebene Vorrichtung 10 weist die Vorrichtung 10b eine Probenkammer 12b zur Aufnahme eines Probentiegels 14b mit einer darauf aufgesetzten Tiegelabdeckung 16b auf, wobei sich die zu analysierende Probe im Inneren des Probentiegels 14b befindet. Die Probenkammer 12b besitzt in einem oberen Bereich der Vorrichtung 10b eine Kammeröffnung 18b, durch welche hindurch zur Vorbereitung der Analyse der Probentiegel 14b in die Probenkammer 12b eingebracht werden kann. Die Vorrichtung 10b weist ferner eine Temperiereinrichtung 20b auf, mittels welcher bei der Analyse der der Probe die Probenkammer 12b gemäß eines vorbestimmten Temperaturprogramms temperiert wird, in dessen Verlauf eine Kammertemperatur im Inneren der Probenkammer 12b verändert und mittels einer Messeinrichtung der Vorrichtung 10b eine Probentemperatur und zusätzlich eine oder mehrere weitere Messgrößen betreffend Eigenschaften der Probe gemessen werden.

Im Unterschied zur Vorrichtung 10 von Fig. 1 ist die Vorrichtung 10b zur Durchführung einer thermischen Analyse der Probe umfassend die Methode der dynamischen Differenzkalorimetrie (DSC) ausgebildet.

In diesem Fall sind in der Probenkammer 12b zwei als "Sensoren" fungierende (mit Themoelementen zur Temperaturmessung ausgestattete) Probentiegelträger 28b-1, 28b-2 vorgesehen, auf denen jeweils ein Probentiegel der beschriebenen Art abgestellt werden kann. Bei der Durchführung der DSC werden gleichzeitig beide Probentiegel bzw. ggf. auch gleichzeitig zwei Proben (z. B. "eigentliche Probe" und "Referenzprobe") einer gemeinsamen Temperierung in der Probenkammer 12b unterzogen. Alternativ zur gleichzeitigen Temperierung von zwei Proben kann der zweite Tiegel während des Verfahrens z. B. auch "leer" (d. h. ohne darin gelagerte Probe bzw. Referenzprobe) eingesetzt werden.

Die Messeinrichtung der Vorrichtung 10b umfasst daher eine Temperaturmesseinrichtung zur Messung der Temperaturen sowohl der eigentlichen Probe (im Probentiegel 14b) als auch der Referenzprobe (bzw. eines "leeren" zweiten Probentiegels).

Jeder der Probentiegelträger 28b-1, 28b-2 dient in der Verwendungssituation der Vorrichtung 10b dazu, einen Probentiegel wie z. B. den dargestellten Probentiegel 14b darauf anordnen zu können, um somit den Probentiegel mitsamt gegebenenfalls beinhalteter Probe (einschließlich "Referenzprobe") in der Probenkammer 12b definiert anzuordnen, und dazu, eine Temperatur an der Unterseite des betreffenden Tiegels bzw. somit die Probentemperatur (im Falle des die Probe enthaltenden Tiegels 14b) zu messen. Hierfür ist an der Oberfläche oder im Inneren jedes Probentiegelträger 28b-1, 28b-2 ein (in der Figur nicht dargestelltes) Thermoelement angeordnet.

Bei der mittels der Vorrichtung 10b durchgeführten dynamischen Differenzkalorimetrie wird im Rahmen der Auswertung eines Messergebnisses (Messdaten) insbesondere ein zeitabhängiger Verlauf einer Differenz der mittels der beiden Sensoren (Probentiegelträger 28b-1, 28b-2) gemessenen Temperaturen ermittelt, insbesondere um damit energetische Effekte und/oder z. B. eine temperaturabhängige spezifische Wärmekapazität der Probe ermitteln zu können. In einer (nicht dargestellten) Weiterbildung der Vorrichtung 10b könnte z. B. auch eine Kombination der DSC mit wenigstens einer weiteren thermoanalytischen Methode wie insbesondere z. B. einer TG (Thermogravimetrische Analyse) vorgesehen sein.

Wie bei der bereits weiter oben beschriebenen Vorrichtung 10 (Fig. 1) sind auch bei der Vorrichtung 10b (Fig. 8) eine Kammerabdeckung 40b und die Stecheinrichtung 42b als eine Abdeckungs/Stech-Einheit 40b, 42b baulich zusammengefasst ausgebildet, welche wieder eine Wandung 46b, ein daran ausgebildetes Führungslager 48b, und eine darin bewegbar gelagerte Nadel 44b aufweist.

Im Unterschied zur Vorrichtung 10 (Fig. 1) ist bei der Vorrichtung 10b (Fig. 8), in Anpassung an die "exzentrische" Position der aufzustechenden Tiegelabdeckung 16b des Probentiegels 14b, das Führungslager 48b und somit die Nadel 44b jedoch nicht im Zentrum der (z. B. kreisrunden) Kammerabdeckung 40b sondern exzentrisch angeordnet.

Zusammenfassend ermöglichen die Erfindung und die beschriebenen Ausführungsbeispiele es, einen luftdicht verschlossenen Probentiegel unter Schutzgasbedingungen in eine Vorrichtung für eine thermische Analyse einer Probe einzubringen und erst dort unter Schutzgasbedingungen zu öffnen. Vorteilhaft wird somit im Rahmen der Probenvorbereitung die Gefahr einer Veränderung der Probe infolge unerwünschter chemischer oder physikalischer Reaktionen mit Bestandteilen der Umgebungsluft drastisch verringert.

## Patentansprüche

1. Vorrichtung (10) für eine thermische Analyse einer Probe, aufweisend
- eine Probenkammer (12) zur Aufnahme eines Probentiegels (14) mit einer zum luftdichten Verschluss des Probentiegels (14) darauf aufgesetzten Tiegelabdeckung (16), in dessen Inneren sich eine zu analysierende Probe befindet, wobei die Probenkammer (12) eine Kammeröffnung (18) zum Einbringen des Probentiegels (14) im verschlossenen Zustand in die Probenkammer (12) besitzt,
- eine Temperiereinrichtung (20) zum Temperieren der Probenkammer (12),
- eine Messeinrichtung zur Messung einer Temperatur der Probe und einer oder mehrerer weiterer Messgrößen,
- eine Gasführungseinrichtung (30, 31, 32) zum Erzeugen einer definierten Gasatmosphäre in der Probenkammer (12), nachdem der Probentiegel (14) im verschlossenen Zustand in die Probenkammer (12) eingebracht wurde,
- eine auf die Kammeröffnung (18) der Probenkammer (12) aufsetzbare Kammerabdeckung (40),
- eine mit einer Nadel (44) ausgestattete Stecheinrichtung (42), die dazu geeignet ist, bei im verschlossenen Zustand in der Probenkammer (12) aufgenommenem Probentiegel (14) und auf die Kammeröffnung (18) aufgesetzter Kammerabdeckung (40) mit der Nadel (44) ein Loch in die Tiegelabdeckung (16) des Probentiegels (14) zu stechen, um den Probentiegel (14) unter der definierten Gasatmosphäre durch das Aufstechen der Tiegelabdeckung (16) zu öffnen.

2. Vorrichtung (10) nach Anspruch 1, wobei die Kammerabdeckung (40) und die Stecheinrichtung (42) als eine Abdeckungs/Stech-Einheit (40, 42) baulich zusammengefasst ausgebildet sind.

3. Vorrichtung (10) nach Anspruch 2, wobei die Vorrichtung (10) eine weitere Kammerabdeckung (40) aufweist, die anstelle der Abdeckungs/Stech-Einheit (40, 42) auf die Kammeröffnung (18) der Probenkammer (12) aufsetzbar ist.

4. Vorrichtung (10) nach Anspruch 2 oder 3, wobei die Abdeckungs/Stech-Einheit (40, 42) aufweist:
- eine zur Abdeckung der Kammeröffnung (18) vorgesehene Wandung (46),
- ein an der Wandung (46) ausgebildetes Führungslager (48) zur bewegbaren Lagerung der Nadel (44), wobei die Nadel (44) sich von einer bei aufgesetzter Abdeckungs/Stech-Einheit (40, 42) der Probenkammer (12) zugewandten Innenseite der Wandung (46) durch die Wandung (46) hindurch bis auf eine bei aufgesetzter Abdeckungs/Stech-Einheit (40, 42) der Probenkammer (12) abgewandten Außenseite der Wandung (46) erstreckt.

5. Vorrichtung (10) nach einem der vorangehenden Ansprüche, wobei die Stecheinrichtung (42) einen mechanischen Anschlag zur Begrenzung einer Einstechtiefe der Nadel (44) aufweist.

6. Vorrichtung (10) nach einem der vorangehenden Ansprüche, wobei die Stecheinrichtung (42) eine mechanische Zentriereinrichtung (60) zur Zentrierung des Probentiegels (14) samt Tiegelabdeckung (16) bezüglich der Nadel (44) aufweist.

7. Vorrichtung (10) nach einem der vorangehenden Ansprüche, wobei in der Probenkammer (12) eine Tiegelaufnahme mit einer Abstellfläche zum darauf Abstellen des Probentiegels (14) ausgebildet ist, und wobei die Tiegelaufnahme verlagerbar ist zwischen einer ersten Stellung zum Abstellen des Probentiegels (14) und Stechen eines Lochs in die Tiegelabdeckung (16) und einer zweiten Stellung zum Durchführen der thermischen Analyse der Probe.

8. Verfahren für eine thermische Analyse einer Probe, die sich im Inneren eines Probentiegels (14) mit einer darauf aufgesetzten Tiegelabdeckung (16) befindet, insbesondere unter Verwendung einer Vorrichtung (10) nach einem der vorangehenden Ansprüche, umfassend folgende Schritte:
- Einbringen des Probentiegels (14) mit der zum luftdichten Verschluss des Probentiegels (14) darauf aufgesetzten Tiegelabdeckung (16) durch eine Kammeröffnung (18) einer Probenkammer (12) in die Probenkammer (12),
- Aufsetzen einer Kammerabdeckung (40) auf die Kammeröffnung (18) der Probenkammer (12) und Erzeugen einer definierten Gasatmosphäre in der Probenkammer (12) mittels einer Gasführungseinrichtung (30, 31, 32), nachdem der Probentiegel (14) im verschlossenen Zustand in die Probenkammer (12) eingebracht wurde,
- Stechen eines Lochs in die Tiegelabdeckung (16) des im verschlossenen Zustand in der Probenkammer (12) aufgenommenen Probentiegels (14) mittels einer Stecheinrichtung (42), um den Probentiegel (14) unter der definierten Gasatmosphäre durch das Aufstechen der Tiegelabdeckung (16) zu öffnen,
- Durchführung der thermischen Analyse der Probe mittels einer Temperiereinrichtung (20) zum Temperieren der Probenkammer (12) und einer Messeinrichtung zur Messung einer Temperatur der Probe und einer oder mehrerer weiterer Messgrößen.

9. Verfahren nach Anspruch 8, wobei die Kammerabdeckung (40) und die Stecheinrichtung (42) als eine Abdeckungs/Stech-Einheit (40, 42) baulich zusammengefasst ausgebildet sind, und wobei nach dem Stechen des Lochs in die Tiegelabdeckung (16) und vor der Durchführung der thermischen Analyse der Probe die Abdeckungs/Stech-Einheit (40, 42) von der Kammeröffnung (18) der Probenkammer (12) abgenommen und durch eine andere Kammerabdeckung ersetzt wird.

10. Verfahren nach Anspruch 9, wobei die Erzeugung der Gasatmosphäre durch eine mittels der Gasführungseinrichtung (30, 31, 32) bewirkte Gasdurchströmung der Probenkammer (12) realisiert wird, deren Strömungsrate während des Ersetzens der Abdeckungs/Stech-Einheit (40, 42) durch die andere Kammerabdeckung höher ist als während der Durchführung der thermischen Analyse.

11. Verwendung eines Verfahrens nach einem der Ansprüche 8 bis 10 zur Durchführung einer oder mehrerer thermischer Analysen einer Probe, ausgewählt aus der Gruppe bestehend aus Thermogravimetrische Analyse, Differentialthermoanalyse und Dynamische Differenzkalorimetrie.

## Claims

1. Device (10) for thermal analysis of a sample, comprising
- a sample chamber (12) for receiving a sample crucible (14) with a crucible cover (16) placed thereon for airtight sealing of the sample crucible (14), inside which there is a sample to be analyzed, wherein the sample chamber (12) has a chamber opening (18) for inserting the sample crucible (14) in its sealed state into the sample chamber (12),
- a temperature controlling means (20) for controlling the temperature of the sample chamber (12),
- a measuring means for measuring the temperature of the sample and one or more other measurement quantities,
- a gas guiding means (30, 31, 32) for generating a defined gas atmosphere in the sample chamber (12) after the sample crucible (14) in its sealed state has been inserted into the sample chamber (12),
- a chamber cover (40) that can be placed on the chamber opening (18) of the sample chamber (12),
- a piercing means (42) having a needle (44) that is suitable for piercing a hole in the crucible cover (16) of the sample crucible (14) with the needle (44) when the sample crucible (14) in its sealed state is received in the sample chamber (12) and the chamber cover (40) is placed on the chamber opening (18), in order to open the sample crucible (14) under the defined gas atmosphere by perforating the crucible cover (16).

2. Device (10) according to claim 1, wherein the chamber cover (40) and the piercing means (42) are structurally combined to form a cover/piercing unit (40, 42).

3. Device (10) according to claim 2, wherein the device (10) has an additional chamber cover (40) which can be placed on the chamber opening (18) of the sample chamber (12) instead of the cover/piercing unit (40, 42).

4. Device (10) according to claim 2 or 3, wherein the cover/piercing unit (40, 42) comprises:
- a wall (46) provided for covering the chamber opening (18),
- a guide bearing (48) formed at the wall (46) for movably supporting the needle (44), wherein the needle (44) extends from an inner side of the wall (46) facing the sample chamber (12) when the cover/piercing unit (40, 42) is placed, through the wall (46) to an outer side of the wall (46) facing away from the sample chamber (12) when the cover/piercing unit (40, 42) is placed.

5. Device (10) according to any one of the preceding claims, wherein the piercing means (42) has a mechanical stop for limiting a piercing depth of the needle (44).

6. Device (10) according to any one of the preceding claims, wherein the piercing means (42) comprises a mechanical centering means (60) for centering the sample crucible (14) together with the crucible cover (16) with respect to the needle (44).

7. Device (10) according to any one of the preceding claims, wherein a crucible receptacle with a support surface for placing the sample crucible (14) thereon is formed in the sample chamber (12), and wherein the crucible receptacle is movable between a first position for placing the sample crucible (14) and piercing a hole in the crucible cover (16) and a second position for performing the thermal analysis of the sample.

8. Method for thermal analysis of a sample which is located inside a sample crucible (14) with a crucible cover (16) placed thereon, in particular using a device (10) according to any one of the preceding claims, comprising the following steps:
- Inserting the sample crucible (14) with the crucible cover (16) placed thereon for airtight sealing of the sample crucible (14) through a chamber opening (18) of a sample chamber (12) into the sample chamber (12),
- Placing a chamber cover (40) on the chamber opening (18) of the sample chamber (12) and generating a defined gas atmosphere in the sample chamber (12) by means of a gas guiding means (30, 31, 32) after the sample crucible (14) in its sealed state has been inserted into the sample chamber (12),
- Piercing a hole in the crucible cover (16) of the sample crucible (14) received in its sealed state in the sample chamber (12) by means of a piercing means (42) in order to open the sample crucible (14) under the defined gas atmosphere by perforating the crucible cover (16),
- Performing the thermal analysis of the sample using a temperature controlling means (20) for controlling the temperature of the sample chamber (12) and a measuring means for measuring a temperature of the sample and one or more other measurement quantities.

9. Method according to claim 8, wherein the chamber cover (40) and the piercing means (42) are structurally combined to form a cover/piercing unit (40, 42), and wherein, after piercing the hole in the crucible cover (16) and before performing the thermal analysis of the sample, the cover/piercing unit (40, 42) is removed from the chamber opening (18) of the sample chamber (12) and replaced by another chamber cover.

10. Method according to claim 9, wherein the gas atmosphere is generated by a gas flow through the sample chamber (12) effected by means of the gas guiding means (30, 31, 32), the flow rate of which during the replacement of the cover/piercing unit (40, 42) with the other chamber cover is higher than during performance of the thermal analysis.

11. Use of a method according to any one of claims 8 to 10 for performing one or more thermal analyses of a sample selected from the group consisting of thermogravimetric analysis, differential thermal analysis, and dynamic differential calorimetry.

## Revendications

1. Dispositif (10) d'analyse thermique d'un échantillon, comprenant
- une chambre d'échantillon (12) pour recevoir un creuset d'échantillon (14) avec un couvercle de creuset (16) placé dessus pour une fermeture hermétique du creuset d'échantillon (14), à l'intérieur de laquelle se trouve un échantillon à analyser, la chambre d'échantillon (12) comportant une ouverture de chambre (18) pour introduire le creuset d'échantillon (14) en état fermé dans la chambre d'échantillon (12),
- un moyen de contrôle de la température (20) pour contrôler la température de la chambre d'échantillon (12),
- un moyen de mesure pour mesurer la température de l'échantillon et une ou plusieurs autres quantités à mesurer,
- un moyen de guidage de gaz (30, 31, 32) pour créer une atmosphère de gaz définie dans la chambre d'échantillon (12) après que le creuset d'échantillon (14) a été introduit à l'état fermé dans la chambre d'échantillon (12),
- un couvercle de chambre (40) pouvant être placé sur l'ouverture de chambre (18) de la chambre d'échantillon (12),
- un moyen de perçage (42) muni d'une aiguille (44), qui est adapté pour percer, à l'aide de l'aiguille (44), un trou dans le couvercle de creuset (16) du creuset d'échantillon (14) lorsque le creuset d'échantillon (14) est placé à l'état fermé dans la chambre d'échantillon (12) et le couvercle de chambre (40) est placé sur l'ouverture de chambre (18), afin d'ouvrir le creuset d'échantillon (14) sous l'atmosphère de gaz définie en perçant le couvercle de creuset (16).

2. Dispositif (10) selon la revendication 1, dans lequel le couvercle de chambre (40) et le moyen de perçage (42) sont combinés de manière structurelle pour former une unité de couvercle/perçage (40, 42).

3. Dispositif (10) selon la revendication 2, dans lequel le dispositif (10) comprend un autre couvercle de chambre (40) qui peut être placé sur l'ouverture de chambre (18) de la chambre d'échantillon (12) au lieu de l'unité de couvercle/perçage (40, 42).

4. Dispositif (10) selon l'une des revendications 2 ou 3, dans lequel l'unité de couvercle/perçage (40, 42) comprend :
- une paroi (46) conçue pour couvrir l'ouverture de chambre (18),
- un palier de guidage (48) formé à la paroi (46) pour supporter l'aiguille (44) de manière mobile, dans lequel l'aiguille (44) s'étend à partir d'un côté intérieur de la paroi (46) faisant face à la chambre d'échantillon (12) lorsque l'unité de couvercle/perçage (40, 42) est en place, à travers la paroi (46) jusqu'à un côté extérieur de la paroi (46) opposé à la chambre d'échantillon (12) lorsque l'unité de couvercle/perçage (40, 42) est en place.

5. Dispositif (10) selon l'une des revendications précédentes, dans lequel le moyen de perçage (42) comprend une butée mécanique pour limiter la profondeur de perçage de l'aiguille (44).

6. Dispositif (10) selon l'une des revendications précédentes, dans lequel le moyen de perçage (42) comprend un moyen de centrage mécanique (60) pour centrer le creuset d'échantillon (14) avec le couvercle de creuset (16) par rapport à l'aiguille (44).

7. Dispositif (10) selon l'une des revendications précédentes, dans lequel un logement de creuset avec une surface d'appui est prévu dans la chambre d'échantillon (12) pour y déposer le creuset d'échantillon (14), et dans lequel le logement de creuset est déplaçable entre une première position pour déposer le creuset d'échantillon (14) et percer un trou dans le couvercle de creuset (16) et une deuxième position pour mis en œuvre l'analyse thermique de l'échantillon.

8. Procédé d'analyse thermique d'un échantillon qui se trouve à l'intérieur d'un creuset d'échantillon (14) avec un couvercle de creuset (16) posé dessus, en particulier en utilisant un dispositif (10) selon l'une des revendications précédentes, comprenant les étapes suivantes :
- Introduire le creuset d'échantillon (14) avec le couvercle de creuset (16) posé dessus pour fermer hermétiquement le creuset d'échantillon (14) à travers une ouverture de chambre (18) d'une chambre d'échantillon (12) dans la chambre d'échantillon (12)
- Poser un couvercle de chambre (40) sur l'ouverture de chambre (18) de la chambre d'échantillon (12) et créer une atmosphère de gaz définie dans la chambre d'échantillon (12) à l'aide d'un moyen de guidage de gaz (30, 31, 32) après que le creuset d'échantillon (14) à l'état fermé a été introduit dans la chambre d'échantillon (12),
- Percer un trou dans le couvercle de creuset (16) du creuset d'échantillon (14) reçu à l'état fermé dans la chambre d'échantillon (12) à l'aide d'un moyen de perçage (42) afin d'ouvrir le creuset d'échantillon (14) sous l'atmosphère de gaz définie en perforant le couvercle de creuset (16),
- Mise en œuvre de l'analyse thermique de l'échantillon à l'aide d'un moyen de contrôle de la température (20) pour contrôler la température de la chambre d'échantillon (12) et d'un moyen de mesure pour mesurer la température de l'échantillon et une ou plusieurs autres quantités à mesurer.

9. Procédé selon la revendication 8, dans lequel le couvercle de chambre (40) et le moyen de perçage (42) sont combinés de manière structurelle pour former une unité de couvercle/perçage (40, 42), et dans lequel, après le perçage du trou dans le couvercle de creuset (16) et avant la mise en œuvre de l'analyse thermique de l'échantillon, l'unité de couvercle/perçage (40, 42) est retirée de l'ouverture de chambre (18) de la chambre d'échantillon (12) et remplacée par un autre couvercle de chambre.

10. Procédé selon la revendication 9, dans lequel la création de l'atmosphère de gaz est réalisée par un flux de gaz à travers la chambre d'échantillon (12) à l'aide du moyen de guidage de gaz (30, 31, 32), dont le débit est plus élevé pendant le remplacement de l'unité de couvercle/perçage (40, 42) par l'autre couvercle de chambre que pendant la mise en œuvre de l'analyse thermique.

11. Utilisation d'un procédé selon l'une des revendications 8 à 10 pour la mise en œuvre d'une ou plusieurs analyses thermiques d'un échantillon, choisies dans le groupe constitué par l'analyse thermogravimétrique, l'analyse thermique différentielle et la calorimétrie différentielle dynamique.
